# EUROPEAN PATENT SPECIFICATION

(11) **EP 3 200 673 B1**
(45) Date of publication and mention of the grant of the patent: **19.06.2024**
(21) Application number: 15778555.1
(22) Date of filing: 30.09.2015
(51) Int. Cl.: A61B 1/307, A61B 1/00, A61B 1/01

(54) **URETEROSCOPE AND ATTACHMENT DEVICE THEREFOR**
URETHROSKOP UND BEFESTIGUNGSVORRICHTUNG DAFÜR
URÉTÉROSCOPE ET DISPOSITIF DE FIXATION ASSOCIÉ

(30) Priority: 02.10.2014 US 201462058710 P
(43) Date of publication of application: 09.08.2017
(73) Proprietor: Boston Scientific Scimed, Inc., Maple Grove, MN 55311 (US)
(72) Inventor: OSKIN, Christopher L., Grafton, MA 01519 (US); BARENBOYM, Michael, Boston, MA 02118 (US)
(74) Representative: Pfenning, Meinig & Partner mbB
(86) International application number: PCT/US2015/053212
(87) International publication number: WO 2016/054202

(56) References cited:
- DE-A1- 3 834 156
- US-A- 5 048 508
- US-A1- 2009 105 543
- US-A1- 2009 306 471

## Description

### Technical Field

Embodiments of the present disclosure relate generally to medical devices used during cystoscopy and ureteroscopy procedures.

### Background

A ureteroscopy is an examination of the ureter or kidney of a patient using a ureteroscope. Ureteroscopes typically include a long, thin, flexible portion that can be inserted through the patient's urethra, bladder, and ureteral orifice connecting the bladder and the ureter. Ureteroscopes typically further include a camera and lighting system to adequately visualize the scope's pathway and the working area within the ureter or kidney. Often, a ureteroscopy procedure involves treating a stone that has lodged in the patient's mid to upper ureter or within a calyx of the kidney.

Currently, before a ureteroscope can be inserted into a patient's ureter, a separate device called a cystoscope is required to examine the bladder and find the ureteral orifice. An examination of the bladder is called a cystoscopy. The cystoscope has a separate camera and lighting system, is often shorter than a ureteroscope, and is typically more rigid than a ureteroscope. Once the ureteral orifice has been located by the cystoscope, a guidewire is inserted into the ureter. The cystoscope is then removed from the patient, leaving the guidewire in place. An access sheath is often then inserted over the guidewire through the urethra, bladder, and into the ureter. The guidewire is removed and the ureteroscope can then be inserted through the access sheath and into the ureter.

The use of two different scopes and accompanying equipment during a ureteroscopy is expensive. Furthermore, the additional steps required to remove the cystoscope from the patient and subsequently insert the ureteroscope through the urethra, bladder, and ureter increases the length of the procedure.

For example, document DE 38 34 156 A1 discloses a rigid medical endoscope having a working channel as introduction aid for flexible endoscopes which is characterised in that the rigid endoscope is constructed such that it is splittable over its entire length through the working channel.

Document US 2009/306471 A1 discloses systems which provide transvesical access to a body cavity (e.g., the peritoneum, the bladder, the ureter, the renal pelvis, or the retroperitoneum).

### SUMMARY

The invention is set out in the appended set of claims. Embodiments of the present disclosure relate to, among other things, a medical device including an ureteroscope an attachment device for a ureteroscope and non-claimed methods for using the attachment device with the ureteroscope. Each of the embodiments disclosed herein may include one or more of the features described in connection with any of the other disclosed embodiments.

In accordance with the invention, a medical device is provided which includes an ureteroscope and an attachment device for the ureteroscope. The ureteroscope includes a handle assembly and a tubular member. The attachment device includes a proximal guide configured to removably connect to the ureteroscope and a distally extending sheath, which is also referred to as the first sheath in the claims and in the following. The first sheath has sufficient rigidity to maintain a generally straight configuration. The first sheath has a proximal end coupled to a distal end of the proximal guide. The first sheath includes an upper lumen and a lower lumen. The upper lumen has an open cross section. The attachment device further includes a second sheath, wherein the second sheath is received within the upper lumen of the first sheath, and a locking feature, which is coupled to a proximal end of the second sheath and configured to connect the second sheath to the first sheath. A distal portion of the tubular member of the ureteroscope is received within the second sheath of the attachment device.

The first sheath is an outer sheath, and the second sheath is an inner sheath configured to fit within the outer (first) sheath. The attachment device may further include one or more of the following features: the upper lumen and the lower lumen of the first sheath may be substantially parallel; alternatively or additionally a coupling feature may connect the guide to the ureteroscope; alternatively or additionally the coupling feature may include an attachment device connecting portion on the attachment device for coupling with a ureteroscope connecting portion on the ureteroscope, and the attachment device connecting portion may be configured to interlock with the ureteroscope connecting portion; alternatively or additionally the guide may include a first opening leading to a first pathway and a second opening leading to a second pathway; alternatively or additionally the first pathway and the second pathway of the guide may converge into a single pathway; alternatively or additionally the single pathway of the guide may be continuous with the lower lumen of the outer sheath alternatively or additionally the inner sheath may be an elongated tube configured to slide within the upper lumen of the outer sheath; alternatively or additionally the locking feature of the attachment device may be removably coupled to the outer sheath; alternatively or additionally the locking feature may form a fluid-tight seal with the coupled inner sheath and outer sheath such that fluid cannot travel proximally through the upper lumen of the outer sheath; and, alternatively or additionally, the locking feature may include a first arm and a second arm that extend along opposite sides of the outer sheath when the locking feature is coupled to the outer sheath.

The elongated tubular member may include an image sensor. The attachment device may be removably coupled to the handle.

As mentioned above, the first sheath is an outer sheath, and the attachment device further includes an inner (second) sheath configured to fit within the outer sheath; the tubular member of the ureteroscope sits within the inner sheath of the attachment device; the medical device includes a locking feature coupled to the proximal end of the inner sheath and removably coupled to the outer sheath; the attachment device further includes a guide; the first sheath includes an upper lumen and a lower lumen; and the upper lumen has an open cross section. The medical device may further include one or more of the following features: the guide may include a first opening leading to a first pathway and a second opening leading to a second pathway.

In an additional example, a non-claimed method for using a ureteroscope with an attachment device is disclosed for illustrative purposes. The method may include coupling an attachment device to a ureteroscope such that the ureteroscope can be used to perform a cystoscopy procedure. The attachment device may include a sheath having a higher durometer than a tubular member of the ureteroscope. The method may further include uncoupling the sheath from the ureteroscope such that the ureteroscope can be used to perform a ureteroscopy procedure.

The method may further include one or more of the following features or steps: the method may further include performing a cystoscopy procedure, wherein the step of performing a cystoscopy procedure may occur after coupling the attachment device to the ureteroscope but before uncoupling the sheath from the ureteroscope; the step of performing a cystoscopy procedure may include locating a patient's ureteral orifice; the sheath may be an outer sheath, and the attachment device may further include an inner sheath; the method may further include inserting the inner sheath of the attachment device into a patient's ureter; the method may further include performing a ureteroscopy procedure; the step of performing a ureteroscopy procedure may occur after uncoupling the sheath from the ureteroscope; and the ureteroscopy procedure may include an examination of at least one of the patient's ureter or kidney.

It is to be understood that both the foregoing general description and the following detailed description are exemplary and explanatory only and are not restrictive of the disclosure. As stated above, the invention is defined by the appended claims.

### BRIEF DESCRIPTION OF THE DRAWINGS

The accompanying drawings, which are incorporated in and constitute a part of this specification, illustrate exemplary embodiments of the present disclosure and together with the description serve to explain the principles of the disclosure.
FIG. 1 illustrates an attachment device coupled to a ureteroscope, according to an exemplary embodiment;
FIG. 2 illustrates components of an attachment device and a ureteroscope, according to an exemplary embodiment;
FIG. 3 illustrates a cross-sectional view of an outer sheath and guide of an attachment device, according to an exemplary embodiment;
FIG. 4 illustrates a cross-sectional view of an outer sheath and inner sheath of an attachment device, according to an exemplary embodiment;
FIG. 5A illustrates an attachment device and a ureteroscope during a cystoscopy procedure, the procedure as such not forming part of the claimed invention;
FIG. 5B illustrates an inner sheath of an attachment device and a ureteroscope during a ureteroscopy procedure, the procedure as such not forming part of the claimed invention; and
FIG. 6 is a flow chart illustrating a non-claimed method for using the attachment device.

### DETAILED DESCRIPTION

### Overview

Embodiments of the present disclosure include an attachment device for a ureteroscope. The attachment device disclosed in this application may allow a medical practitioner to perform both a cystoscopy procedure and a ureteroscopy procedure using the same camera, lighting, and other equipment. The related non-claimed methods disclosed in this application may allow a medical practitioner to eliminate certain steps from ureteroscopy procedures.

### Exemplary Embodiments

Reference will now be made in detail to exemplary embodiments of the present disclosure.

The terms "proximal" and "distal" are used herein to refer to the relative positions of the components of an exemplary medical device. When used herein, "proximal" refers to a position relatively closer to a user using the medical device. In contrast, "distal" refers to a position relatively farther away from the user using the medical device.

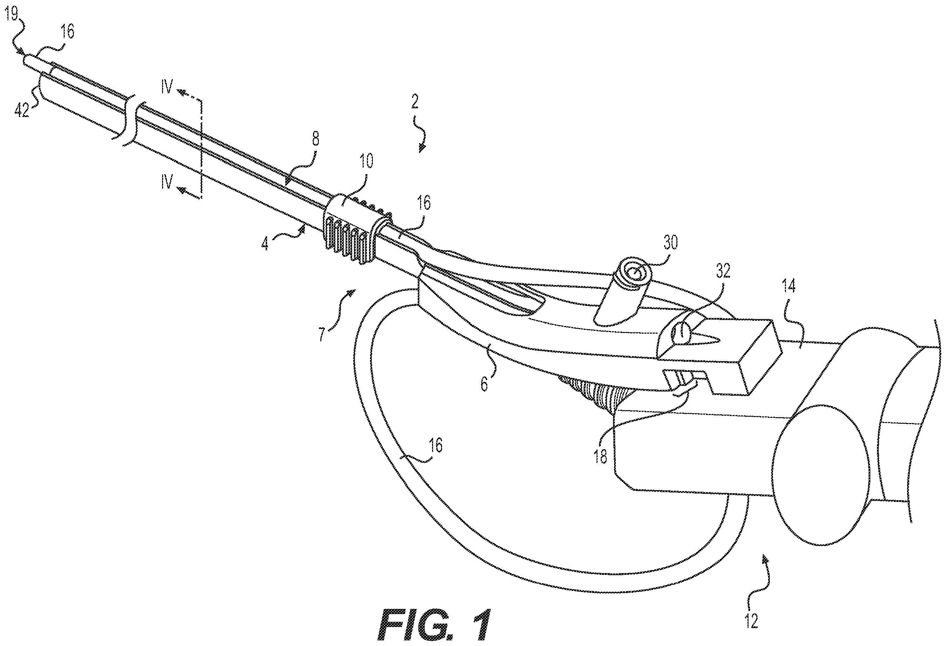

FIG. 1 illustrates an attachment device 2 removably coupled to a ureteroscope 12 via a coupling feature 18. The attachment device 2 includes an outer (first) sheath 4, a guide 6, an inner (second) sheath 8, and a locking feature 10. In this embodiment, the outer sheath 4 and the guide 6 may be coupled together to form a single piece, referred to herein as the rigid portion 7, although the outer sheath 4 and the guide 6 may be separable in other embodiments. The outer sheath 4 includes two lumens that will be described in greater detail below, but in general, one lumen may hold tools and irrigation fluids and the other lumen may hold the inner sheath 8. The guide 6 of the rigid portion 7 may include a first opening 30 and a second opening 32. The inner sheath 8 is an elongated tube with its proximal end coupled to the locking feature 10. The locking feature 10 may be removably coupled to the outer sheath 4.

Durometer is a measure of the hardness of a material, and in one embodiment, the outer sheath 4 may have a higher durometer than the inner sheath 8. In other words, the outer sheath 4 may be more rigid than the inner sheath 8. Because the inner sheath 8 may have a lower durometer than the outer sheath 4 in this embodiment, the inner sheath 8 may be more flexible than the outer sheath 4. Additionally or alternatively, the outer sheath 4 may have a higher durometer than a tubular member 16 of a ureteroscope.

The outer sheath 4 has sufficient rigidity to maintain a generally straight configuration. In one embodiment, the generally straight configuration may be maintained during a cystoscopy procedure in which the outer sheath 4 is being used to navigate a camera and other tools through a patient's bladder. The rigidity of the outer sheath 4 may allow the practitioner to control, for example, the distal end of a tubular member 16 of a ureteroscope 12. The tubular member 16 by itself may not have sufficient rigidity to maintain a generally straight configuration during navigation through a patient's bladder. In one embodiment, maintaining a generally straight configuration may mean that during normal use, the distal end 42 of the outer sheath 4 stays within 10 degrees (inclusive) of an axis extending distally from the proximal end of the outer sheath 4 (e.g., where the outer sheath 4 connects to guide portion 6).

Still referring to FIG. 1, the ureteroscope 12 includes a handle assembly 14 and a tubular member 16. In this embodiment, the proximal end of the tubular member 16 may be connected to the handle assembly 14. A distal portion of the tubular member 16 sits within the inner sheath 8 of the attachment device 2. A middle portion of the tubular member 16 may form a coil. The ureteroscope 12 may further include a variety of other components necessary for viewing the interior of a body, such as camera and lighting equipment. In one embodiment, an image sensor 19 and a light may be located near the distal end of the tubular member 16. In some embodiments, optical fiber cables are used to transmit light from the distal end of tubular member 16 to a camera located elsewhere. The camera may be coupled to the ureteroscope handle 14, for example. The tubular member 16 may also include one or more working channels for tools and irrigation. Channels in the tubular member 16 may also hold electrical wires and cables connecting the image sensor 19 and light to a power source and other processing and control equipment, along with mechanical cables to permit maneuverability of the tubular member 16.

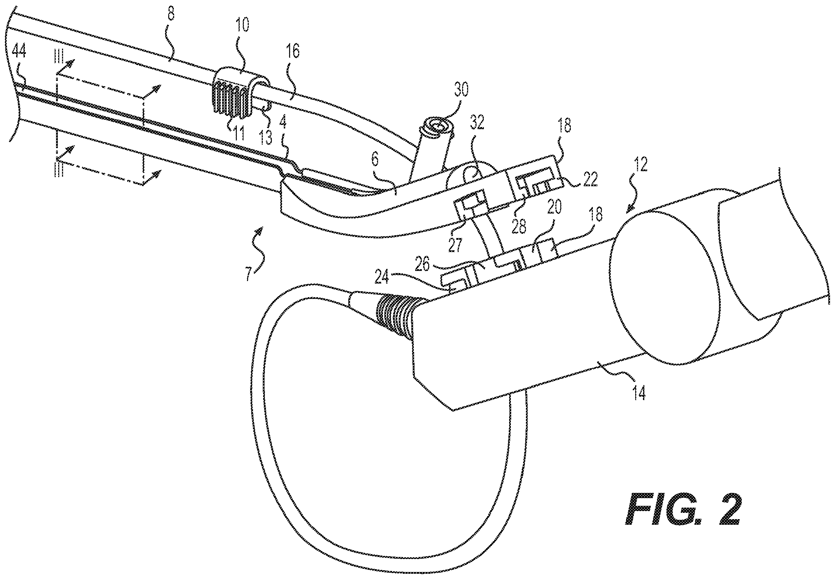

FIG. 2 illustrates the attachment device 2 and the ureteroscope 12 of FIG. 1 with certain components separated. In FIG. 2, the locking feature 10 is uncoupled from the outer sheath 4, and the rigid portion 7 is uncoupled from the handle assembly 14 of the ureteroscope 12. However, as in FIG. 1, the tubular member 16 of the ureteroscope 12 is shown sitting within the inner sheath 8 of the attachment device 2.

The coupling feature 18, which may allow the rigid portion 7 to be removably coupled to the ureteroscope 12, may include a ureteroscope connecting portion 20 and an attachment device connecting portion 22. The ureteroscope connecting portion 20 may include receiving slots 24, 26. The receiving slots may be rectangular, such as slot 24, or "L" shaped, such as slot 26. The attachment device connecting portion 22 of the coupling feature 18 may include protrusions 27, 28. The protrusions 27, 28 may be configured to fit within the receiving slots 24, 26 to securely couple the guide 6 to the handle assembly 14. In other embodiments, the coupling feature 18 may be any other mechanism suitable for coupling the attachment device 2 to the ureteroscope 12.

Still referring to FIG. 2, the locking feature 10, which is coupled to the inner sheath 8, may have a U-shaped cross section. The locking feature 10 may be connected to the inner sheath 8 at the portion of the inner sheath 8 that would otherwise be exposed by the opening in the upper lumen 44 of the outer sheath 4 (when the inner sheath 8 sits within the upper lumen 44 of the outer sheath). The locking feature 10 may include a first arm 11 and a second arm 13 that extend along opposite sides of the outer sheath 4 when the locking feature 10 is coupled to the outer sheath 4 (as shown in FIG. 1). The first arm 11 and second arm 13 may exert a radially inward force to create a friction fit with the outer sheath 4. In another embodiment, the locking feature 10 and the exterior of the outer sheath 4 may be coupled via a ratchet mechanism that allows the locking feature 10 to slide in one direction relative to the outer sheath 4 unless unlocked by a user. The ratchet mechanism may include teeth on the outer sheath 4 and a pawl within the locking feature 10.

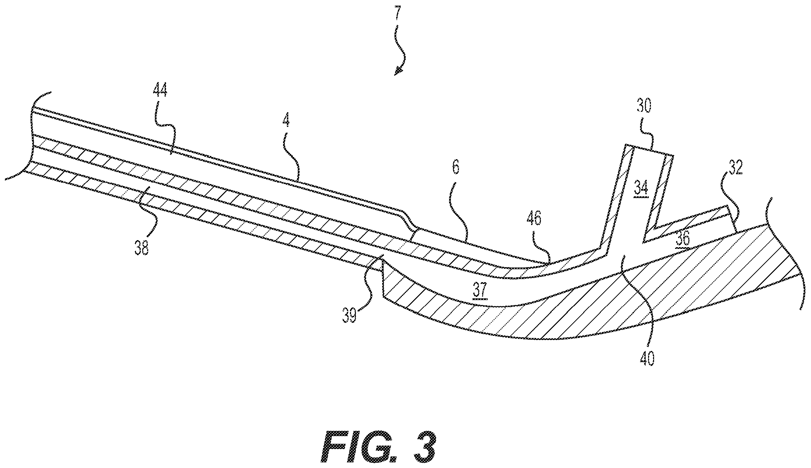

FIG. 3 illustrates a cross section of the rigid portion 7, including the outer sheath 4 and the guide 6, along plane III-III of FIG. 2. As in FIG. 2, the inner sheath 8 and the tubular member 16 have been removed from the outer sheath 4. The first opening 30 of the guide 6 may lead to a first pathway 34. The second opening 32 of the guide 6 may lead to a second pathway 36. In one embodiment, the first opening 30 and the second opening 32 may be Luer connections for forming fluid-tight connections between the guide 6 and other tubes or devices. Pathways 34 and 36 may converge at convergence point 40 to form a single pathway 37. Single pathway 37 may run from convergence point 40 to the intersection of the guide 6 and the outer sheath 4, at connection point 39. The single pathway 37 of the guide 6 may be continuous with the lower lumen 38 of the outer sheath 4.

Lower lumen 38 of the outer sheath 4 may begin at connection point 39 and run to the distal tip 42 (see FIG. 1) of the outer sheath 4. Outer sheath 4 may further include an upper lumen 44 that may run substantially parallel to the lower lumen 38 along the outer sheath 4. The upper lumen 44 may begin on the guide 6 at location 46 and may run distally along the guide 6 and the outer sheath 4 to the distal tip 42 of the outer sheath 4. The upper lumen 44 may be configured to receive the inner sheath 8, which holds the tubular member 16 of the ureteroscope 12. In an alternative embodiment, the outer sheath 4 includes one, three, or more lumens that may hold one or more of the inner sheath 8, the tubular member 16 of the ureteroscope 12, working tools, and irrigation.

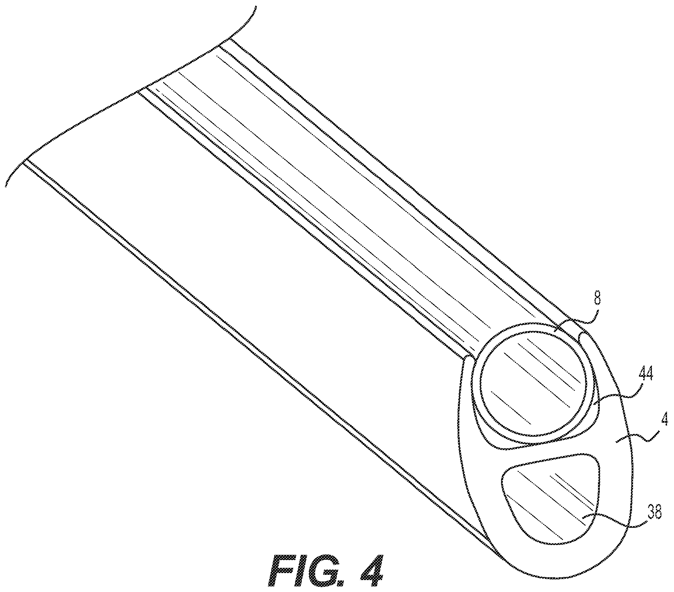

FIG. 4 is a cross-sectional view of outer sheath 4 and inner sheath 8, taken along plane IV-IV of FIG. 1. For simplicity, the tubular member 16 of the ureteroscope 12 is not shown in FIG. 4, although the tubular member 16 sits within the inner sheath 8 during a cystoscopy or ureteroscopy procedure. The lower lumen 38 of the outer sheath 4 may have an enclosed, D-shaped cross section. The upper lumen 44 has an open, e.g. U-shaped, cross section. The upper lumen 44 may have other shapes or may have a variable cross section. The inner sheath 8 sits within the upper lumen 44. As can be seen in FIG. 4, the inner sheath 8 is sized such that it can fit within the upper lumen 44. The inner sheath 8 may be further sized such that a medical practitioner can slide the inner sheath 8 within the upper lumen 44. In an alternative embodiment not forming part of the claimed invention, the inner sheath 8 is placed within the lower lumen 38. In this illustrative example, the various components of the attachment device 2 may be modified to accommodate the alternative placement of the inner sheath 8. The locking feature may be realized by other embodiments, such as, for example, a configuration of a narrow distance between the walls of the upper lumen such that the inner sheath may be pinched, clipped in, or otherwise held in place by frictional forces. Furthermore, at least one example of a locking feature may be integrally formed in the wall of the upper lumen, narrowing a portion of the upper lumen. Alternatively or additionally, such feature may comprise a resilient material, deformable material, spring, or compressive element associated with a resilient material, deformable material, or spring. Such compressive element may be provided with a geometry configured to match or engage the outer diameter of the inner sheath or tubular member.

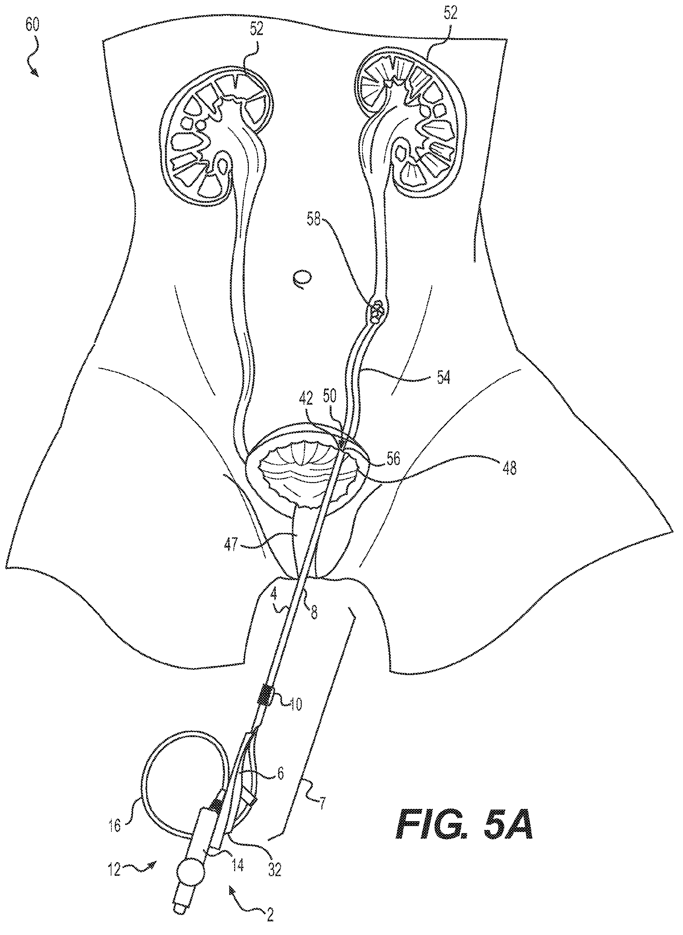

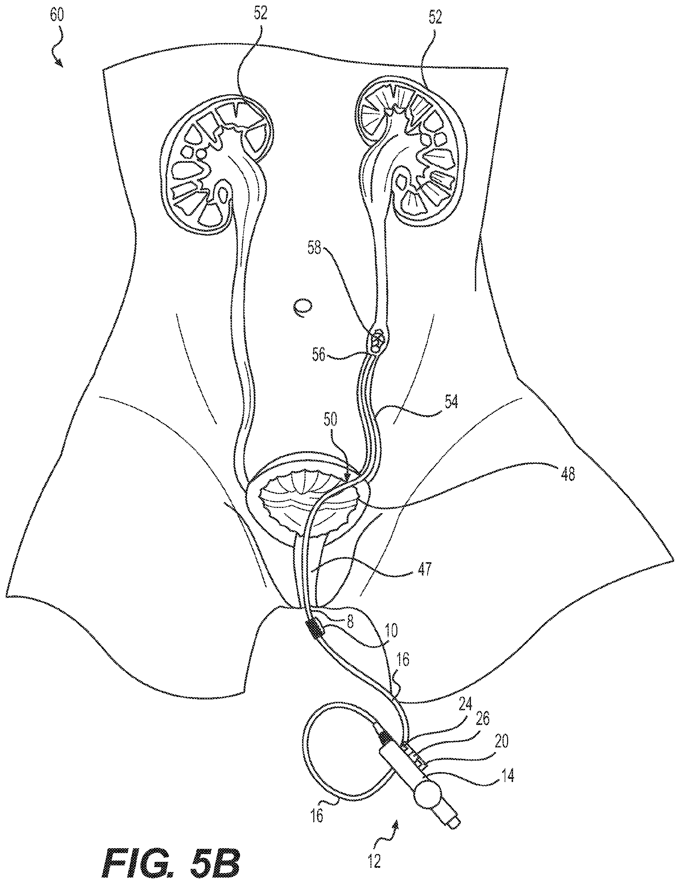

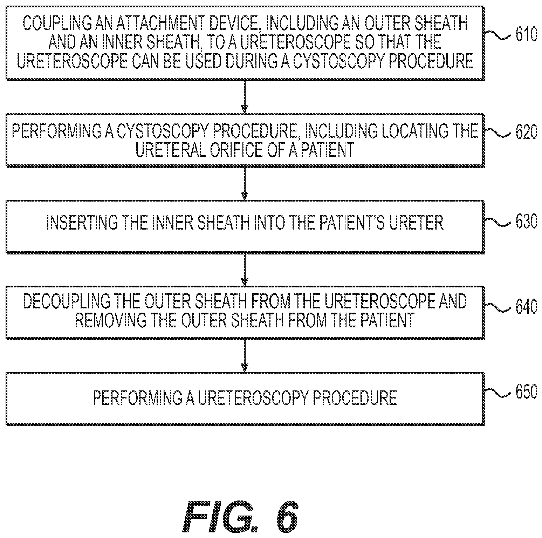

FIGs. 5A, 5B, and 6 will be referenced, in conjunction with FIGs. 1-4, to describe an exemplary non-claimed method for using the attachment device 2. In general, the attachment device 2 may be coupled to the ureteroscope 12 during an examination of the bladder 48 of a patient 60, including to locate the ureteral orifice 50. This portion of the procedure - examining the bladder and locating the ureteral orifice - is called a cystoscopy. During a later stage of the procedure, the rigid portion 7 may be uncoupled from the inner sheath 8 and the handle assembly 14 and removed from the patient 60, while the inner sheath 8 and the tubular member 16 remain in the patient 60. The ureteroscope 12 may then be used during a ureteroscopy procedure to examine the patient's ureter 54 or kidney 52.

Referring to FIG. 6, during a first step 610 of an exemplary non-claimed method for using the attachment device 2, the attachment device 2 may be coupled to a ureteroscope 12. The guide 6 of the attachment device 2 and the handle assembly 14 of the ureteroscope 12 may be coupled using coupling feature 18 (FIG. 2). In this embodiment, the protrusions 27, 28 of the attachment device connecting portion 22 may be aligned with the corresponding slots 24, 26 of the ureteroscope connecting portion 20 and pushed together. The rigid portion 7 may then be pulled proximally (or the ureteroscope handle assembly 14 pushed distally) to slide the protrusions 27, 28 into the slots 24, 26 such that the two connecting portions 20, 22 interlock to couple the attachment device 2 to the ureteroscope 12. It can be seen in FIG. 2 that the attachment device connecting portion 22 may also include slots that receive corresponding protrusions of the ureteroscope connecting portion 20 such that the two connecting portions 20, 22 interlock.

FIG. 5A illustrates the attachment device 2 coupled to the ureteroscope 12 such that the ureteroscope 12 can be used during a cystoscopy procedure. During a cystoscopy procedure, a medical practitioner may examine the bladder 48 and locate the ureteral orifice 50 (FIG. 6, Step 620). To obtain access to the bladder 48, the outer sheath 4, which contains the inner sheath 8 and the tubular member 16, is inserted into the patient's urethra 47 and into the bladder 48. The outer sheath 4 of the rigid portion 7 has a high enough durometer (i.e., is sufficiently rigid) to allow for control of the distal tip 42 of the outer sheath 4 when the distal tip 42 is in the bladder 48. The image sensor 19 of tubular member 16 may be located near the distal tip 42 of the outer sheath 4 when the medical practitioner is examining the bladder wall. The rigidity of the outer sheath 4 allows a medical practitioner to effectively and efficiently navigate a ureteroscope 12 across the bladder to locate the ureteral orifice 50. Conventionally, a separate cystoscope would be required to examine the bladder 48 and locate the ureteral orifice 50.

Once the ureteral orifice 50 is located, the practitioner may insert a guidewire into the ureter 54, as shown in FIG. 5A. The guidewire may be inserted distally through opening 32 of the guide 6, along pathway 36, through lower lumen 38, and into the ureter 54. The guidewire may be pushed distally to the kidney 52. In one embodiment, the second opening 32 of the guide 6 may be used to receive working tools, such as the guidewire, and the first opening 30 may be used to receive fluid for irrigating the working site near the distal tip 42 of the outer sheath 4. The enclosed configuration of lower lumen 38 may allow fluid and tools to be contained within the lower lumen 38 without the use of an additional sheath.

Once the guidewire is in place in the ureter 54, the inner sheath 8 may be pushed distally over the guidewire and into the ureter 54 (Step 630). To move the inner sheath 8 distally, the locking feature 10 may be squeezed or otherwise unlocked by the practitioner and moved distally relative to the outer sheath 4. The locking feature 10 may remain coupled to the inner sheath 8. Therefore, in this embodiment, when the locking feature 10 is pushed distally, the distal end 56 of the inner sheath 8 may move distally into the ureter 54 (see FIG. 5B). FIG. 5B illustrates the distal end 56 of the inner sheath 8 about halfway up the ureter 54, adjacent to a ureteral stone 58. The distal end 56 may be pushed into the ureteral stone 58 such that tools placed through the inner sheath 8 can be used to break apart the ureteral stone 58. The distal end 56 of the inner sheath 8 may also be pushed farther up the ureter 54 to reach stones in the upper ureter or kidney. In another embodiment, a guidewire is not inserted into the ureter 54 and the inner sheath 8 is pushed directly from the bladder 48 into the ureter 54.

Use of the inner sheath 8 of the attachment device may eliminate certain steps from a conventional ureteroscopy procedure that uses both a cystoscope and a ureteroscope. During these conventional ureteroscopy procedures, the cystoscope used to find the ureteral orifice would be removed from the patient and an access sheath would be inserted separately over a guidewire through the urethra, bladder, and ureter. However, when using the inner sheath 8 as part of the attachment device 2, an inner sheath 8 may be inserted into the patient with the outer sheath 4 and can simply be pushed forward from the bladder 48 into the ureter 54. This can shorten the medical procedure, saving time and expense, and spares the patient from the potential damage of having an access sheath pushed separately through the urethra and bladder.

With the inner sheath 8 in place, the outer sheath 4 of the rigid portion 7 may be uncoupled from the ureteroscope 12 and removed from the patient 60 (Step 640). FIG. 5B illustrates the inner sheath 8 within the ureter 54 after the outer sheath 4 has been uncoupled from the ureteroscope 12 and removed from the patient 60. In one embodiment, removal of the outer sheath 4 may involve separating the ureteroscope connecting portion 20 and the attachment device connecting portion 22 of the coupling feature 18. Separating the two connecting portions 20, 22 may be accomplished by sliding the guide 6 and the ureteroscope handle 14 relative to each other (e.g., by sliding the handle 14 proximally) and then pulling the guide 6 and the handle 14 away from each other, as shown in FIG. 2. The locking feature 10 can then be squeezed or otherwise unlocked, and the rigid portion 7 pulled proximally until the outer sheath 4 has been removed from the patient 60. Thus, the outer sheath 4 may slide out of the patient over the inner sheath 8, while the inner sheath 8 remains in the patient. During movement of the outer sheath 4 with respect to the inner sheath 8, the locking feature 10 forms a fluid-tight seal with the portion of the outer sheath 4 facing towards the interior of upper lumen 44. The fluid-tight seal between the locking feature 10 and the coupled inner sheath 8 and outer sheath 4 may help prevent bodily fluids from flowing proximally through the upper lumen 44 of the outer sheath 4.

In one embodiment, during removal of the rigid portion 7, the camera, lighting, and other equipment contained within the tubular member 16 of the ureteroscope may remain generally in the bladder 48, although contained within the inner sheath 8. Once the rigid portion 7 has been removed, the practitioner may uncoil the tubular member 16 and push it, along with its interior equipment, distally through the inner sheath 8. The image sensor 19 may then be used to perform a ureteroscopy procedure (i.e., an examination of the ureter 54) (Step 650) or an examination of the kidney 52. In addition, working tools may be inserted through a lumen within the tubular member 16 to treat a ureteral stone 58 or a kidney stone. In this manner, the medical practitioner may be able to perform all necessary steps of a ureteroscopy procedure with the use of a single scope.

Alternatively, in an example not forming part of the claimed invention, the attachment device 2 may include an outer sheath 4 but no inner sheath. The outer sheath 4 of this embodiment may have one, two, or more lumens, and may be structured and used with a ureteroscope in a similar manner as described in connection with other embodiments of the attachment device 2 described herein. In one example not forming part of the claimed invention, the outer sheath 4 has two lumens that are both enclosed, instead of the open U-shaped upper lumen 44 shown in FIG. 4. The attachment device 2 including an outer sheath 4 but no inner sheath may include a coupling feature 18 to removably couple the outer sheath 4 to a ureteroscope 12 and may further include other combinations of features described herein.

When there is no inner sheath as part of the attachment device 2, the tubular member 16 of the ureteroscope 12 may be held directly within the upper or lower lumen of the outer sheath 4 (also sometimes referred to herein as "sheath 4"). Similar to other embodiments, the sheath 4 of this embodiment may have a durometer that is higher than the durometer of the tubular member 16 of the ureteroscope 12. The higher durometer of the sheath 4 may allow a medical practitioner to effectively and efficiently navigate the tubular member 16 of the ureteroscope 12 through a patient's bladder 48. As described above, the sheath 4 may have sufficient rigidity to maintain a generally straight configuration.

Similar to other embodiments disclosed herein, when using an attachment device 2 without an inner sheath 8, the attachment device 2, including the sheath 4, may be coupled to a ureteroscope during a cystoscopy procedure. After the cystoscopy procedure, the sheath 4 may be uncoupled from the ureteroscope 12 and removed from the patient so that the ureteroscope can be used to perform a ureteroscopy procedure. In one embodiment, the medical practitioner may remove the tubular member 16 and the sheath 4 from the patient after the cystoscopy procedure, leaving a guidewire through the urethra, bladder, and into the ureter. The practitioner may then insert a separate access sheath over the guidewire and into the ureter 54. The tubular member 16 can then be reinserted through the access sheath to perform a ureteroscopy procedure. In another embodiment, the sheath 4 can be removed from the patient while leaving the tubular member 16 within the patient and then performing a ureteroscopy procedure. Whether the attachment device 2 has a separate inner sheath 8 or not, the attachment device 2 may allow the same ureteroscope 12 to be used both during portions of a procedure that require a more rigid portion to navigate through a patient and during portions of the procedure that require a less rigid portion to navigate through the patient.

In a further embodiment, the attachment device 2 may be disposable. A disposable attachment device 2 may save a practitioner the time and expense that would otherwise be required to sterilize a separate cystoscope (for locating the ureteral orifice during a ureteroscopy procedure) between uses.

While principles of the present disclosure are described herein with reference to illustrative embodiments for particular applications, it should be understood that the disclosure is not limited thereto. Those having ordinary skill in the art and access to the teachings provided herein will recognize additional modifications, applications, embodiments, and substitution of equivalents that fall within the scope of the embodiments described herein. Accordingly, the invention is not to be considered as limited by the foregoing description.

## Claims

1. A medical device including an ureteroscope (12) and an attachment device (2) for the ureteroscope (12), the ureteroscope (12) including a handle assembly (14) and a tubular member (16), the attachment device (2) comprising:
a proximal guide (6) configured to removably connect to the ureteroscope (12); and
a distally extending first sheath (4) having a proximal end coupled to a distal end of the proximal guide (6), wherein the first sheath (4) includes an upper lumen (44) and a lower lumen (38), wherein the upper lumen (44) has an open cross section;
a second sheath (8), wherein the second sheath (8) is received within the upper lumen (44) of the first sheath (4); and
a locking feature (10) coupled to a proximal end of the second sheath (8) and configured to connect the second sheath (8) to the first sheath (4),
wherein the first sheath (4) has sufficient rigidity to maintain a generally straight configuration, and wherein a distal portion of the tubular member (16) of the ureteroscope (12) is received within the second sheath (8) of the attachment device (2).

2. The medical device of claim 1, wherein the upper lumen (44) and the lower lumen (38) are substantially parallel.

3. The medical device of any one of the preceding claims, further comprising a coupling feature (18) to connect the guide (6) to the ureteroscope (1).

4. The medical device of claim 3, wherein the coupling feature (18) includes an attachment device connecting portion (22) on the attachment device (2) for coupling with a ureteroscope connecting portion (20) on the ureteroscope (1), and the attachment device connecting portion (22) is configured to interlock with the ureteroscope connecting portion (20).

5. The medical device of any of the preceding claims, wherein the guide (6) includes a first opening (30) leading to a first pathway (34) and a second opening (32) leading to a second pathway (36).

6. The medical device of claim 5, wherein the first pathway (34) and the second pathway (36) converge into a single pathway (37).

7. The medical device of claim 6, including the features of any of claims 2 or 3, wherein the single pathway (37) of the guide (6) is continuous with the lower lumen (38) of the first sheath (4).

8. The medical device of any of the preceding claims, wherein the locking feature (10) has a U-shaped cross section.

9. The medical device of any one of the preceding claims, wherein the locking feature (10) forms a fluid-tight seal with the coupled second sheath (8) and first sheath (4) such that fluid cannot travel proximally through the upper lumen (44) of the first sheath (4).

10. The medical device of any of the preceding claims, wherein the locking feature (10) includes a first arm (11) and a second arm (13) that extend along opposite sides of the first sheath (4) when the locking feature (10) is coupled to the first sheath (4).

11. The medical device of any one of the preceding claims, wherein a proximal end of the tubular member (16) of the ureteroscope (12) is connected to the handle assembly (14) of the ureteroscope (12).

## Patentansprüche

1. Medizinische Vorrichtung mit einem Ureteroskop (12) und einer Befestigungsvorrichtung (2) für das Ureteroskop (12), wobei das Ureteroskop (12) eine Griffbaugruppe (14) und ein schlauchförmiges Element (16) aufweist und die Befestigungsvorrichtung (2) umfasst:
eine proximale Führung (6), die eingerichtet ist, abnehmbar mit dem Ureteroskop (12) verbunden werden zu können; und
eine sich distal erstreckende erste Hülle (4) mit einem proximalen Ende, das mit einem distalen Ende der proximalen Führung (6) verbunden ist, wobei die erste Hülle (4) ein oberes Lumen (44) und ein unteres Lumen (38) aufweist, wobei das obere Lumen (44) einen offenen Querschnitt hat;
eine zweite Hülle (8), wobei die zweite Hülle (8) innerhalb des oberen Lumens (44) der ersten Hülle (4) aufgenommen ist; und
ein Verriegelungselement (10), das mit einem proximalen Ende der zweiten Hülle (8) verbunden ist und eingerichtet ist, die zweite Hülle (8) mit der ersten Hülle (4) zu verbinden,
wobei die erste Hülle (4) eine ausreichende Steifigkeit aufweist, um eine im Allgemeinen gerade Konfiguration beizubehalten, und wobei ein distaler Abschnitt des schlauchförmigen Elements (16) des Ureteroskops (12) innerhalb der zweiten Hülle (8) der Befestigungsvorrichtung (2) aufgenommen ist.

2. Medizinische Vorrichtung nach Anspruch 1, wobei das obere Lumen (44) und das untere Lumen (38) im Wesentlichen parallel sind.

3. Medizinische Vorrichtung nach einem der vorhergehenden Ansprüche, die ferner eine Kupplungseinrichtung (18) zur Verbindung der Führung (6) mit dem Ureteroskop (1) umfasst.

4. Medizinische Vorrichtung nach Anspruch 3, wobei die Kopplungseinrichtung (18) einen Befestigungsvorrichtungs-Verbindungsteil (22) an der Befestigungsvorrichtung (2) zum Koppeln mit einem Ureteroskop-Verbindungsteil (20) am Ureteroskop (1) umfasst und der Befestigungsvorrichtungs-Verbindungsteil (22) so konfiguriert ist, dass er mit dem Ureteroskop-Verbindungsteil (20) verriegelt.

5. Medizinische Vorrichtung nach einem der vorhergehenden Ansprüche, wobei die Führung (6) eine erste Öffnung (30), die zu einem ersten Durchgang (34) führt, und eine zweite Öffnung (32), die zu einem zweiten Durchgang (36) führt, aufweist.

6. Medizinische Vorrichtung nach Anspruch 5, wobei der erste Durchgang (34) und der zweite Durchgang (36) in einen einzigen Durchgang (37) zusammenlaufen.

7. Medizinische Vorrichtung nach Anspruch 6, die die Merkmale eines der Ansprüche 2 oder 3 aufweist, wobei der einzelne Durchgang (37) der Führung (6) mit dem unteren Lumen (38) der ersten Hülle (4) durchgängig ist.

8. Medizinprodukt nach einem der vorhergehenden Ansprüche, wobei das Verriegelungselement (10) einen U-förmigen Querschnitt aufweist.

9. Medizinische Vorrichtung nach einem der vorhergehenden Ansprüche, wobei das Verriegelungsmerkmal (10) eine flüssigkeitsdichte Abdichtung mit der gekoppelten zweiten Hülle (8) und der ersten Hülle (4) bildet, so dass keine Flüssigkeit proximal durch das obere Lumen (44) der ersten Hülle (4) gelangen kann.

10. Medizinische Vorrichtung nach einem der vorhergehenden Ansprüche, wobei das Verriegelungselement (10) einen ersten Arm (11) und einen zweiten Arm (13) aufweist, die sich entlang gegenüberliegender Seiten der ersten Hülle (4) erstrecken, wenn das Verriegelungselement (10) mit der ersten Hülle (4) verbunden ist.

11. Medizinische Vorrichtung nach einem der vorhergehenden Ansprüche, wobei ein proximales Ende des rohrförmigen Elements (16) des Ureteroskops (12) mit der Griffanordnung (14) des Ureteroskops (12) verbunden ist.

## Revendications

1. Dispositif médical incluant un urétéroscope (12) et un dispositif de fixation (2) pour l'urétéroscope (12), l'urétéroscope (12) incluant un assemblage de poignée (14) et un élément tubulaire (16), le dispositif de fixation (2) comprenant :
un guide proximal (6) configuré pour être connecté de façon amovible à l'urétéroscope (12) ; et
une première gaine étendue de façon distale (4) comportant une extrémité proximale couplée à une extrémité distale du guide proximal (6), dans lequel la première gaine (4) inclut une lumière supérieure (44) et une lumière inférieure (38), dans lequel la lumière supérieure (44) présente une section en coupe transversale ouverte ;
une seconde gaine (8), dans lequel la seconde gaine (8) est reçue à l'intérieur de la lumière supérieure (44) de la première gaine (4) ; et
une caractéristique de blocage (10) couplée à une extrémité proximale de la seconde gaine (8) et configurée pour connecter la seconde gaine (8) à la première gaine (4),
dans lequel la première gaine (4) présente une rigidité suffisante pour conserver une configuration généralement rectiligne, et dans lequel une partie distale de l'élément tubulaire (16) de l'urétéroscope (12) est reçue à l'intérieur de la seconde gaine (8) du dispositif de fixation (2).

2. Dispositif médical selon la revendication 1, dans lequel la lumière supérieure (44) et la lumière inférieure (38) sont sensiblement parallèles.

3. Dispositif médical selon l'une quelconque des revendications précédentes, comprenant en outre une caractéristique de couplage (18) pour connecter le guide proximal (6) à l'urétéroscope (1).

4. Dispositif médical selon la revendication 3, dans lequel la caractéristique de couplage (18) inclut une partie de connexion de dispositif de fixation (22) sur le dispositif de fixation (2) pour un couplage avec une partie de connexion d'urétéroscope (20) sur l'urétéroscope (1), et la partie de connexion de dispositif de fixation (22) est configurée pour réaliser un inter-blocage avec la partie de connexion d'urétéroscope (20).

5. Dispositif médical selon l'une quelconque des revendications précédentes, dans lequel le guide proximal (6) inclut une première ouverture (30) qui débouche sur un premier passage (34) et une seconde ouverture (32) qui débouche sur un second passage (36).

6. Dispositif médical selon la revendication 5, dans lequel le premier passage (34) et le second passage (36) convergent selon un unique passage (37).

7. Dispositif médical selon la revendication 6, incluant les caractéristiques selon l'une quelconque des revendications 2 ou 3, dans lequel l'unique passage (37) du guide proximal (6) est en continuité avec la lumière inférieure (38) de la première gaine (4).

8. Dispositif médical selon l'une quelconque des revendications précédentes, dans lequel la caractéristique de blocage (10) présente une section en coupe transversale en forme de U.

9. Dispositif médical selon l'une quelconque des revendications précédentes, dans lequel la caractéristique de blocage (10) forme un joint étanche au fluide avec les seconde (8) et première (4) gaines couplées de sorte que du fluide ne peut pas se déplacer de façon proximale au travers de la lumière supérieure (44) de la première gaine (4).

10. Dispositif médical selon l'une quelconque des revendications précédentes, dans lequel la caractéristique de blocage (10) inclut un premier bras (11) et un second bras (13) qui sont étendus le long de côtés opposés de la première gaine (4) lorsque la caractéristique de blocage (10) est couplée à la première gaine (4).

11. Dispositif médical selon l'une quelconque des revendications précédentes, dans lequel une extrémité proximale de l'élément tubulaire (16) de l'urétéroscope (12) est connectée à l'assemblage de poignée (14) de l'urétéroscope (12).
